# FASCICULE DE BREVET EUROPEEN

(11) **EP 0 835 650 B1**
(45) Date de publication et mention de la délivrance du brevet: **21.07.1999**
(21) Numéro de dépôt: 97402150.3
(22) Date de dépôt: 17.09.1997
(51) Int. Cl.: A61K 7/48

(54) **Utilisation de dérivés d'oxazolidinone comme agents anti-pénétrants dans une composition cosmétique et/ou dermatologique**
Verwendung von Oxazolidinon-Derivaten als penetrations-verhindernde Mittel in einer kosmetischen und/oder dermatologischen Zusammensetzung
Use of oxazolidinon derivatives as antipenetrant agents in a cosmetic and/or dermatologic composition

(30) Priorité: 14.10.1996 FR 9612509
(43) Date de publication de la demande: 15.04.1998
(73) Titulaire: L'OREAL, 75008 Paris (FR)
(72) Inventeur: Philippe, Michel, 91320 Wissous (FR); Tuloup, Rémy, 75014 Paris (FR); Morancais, Jean-Luc, 77230 Ozoir la Ferriere (FR)
(74) Mandataire: Tezier Herman, Béatrice

(56) Documents cités:
- EP-A- 0 391 273
- WO-A-90/00407
- FR-A- 2 379 283

## Description

L'invention se rapporte à l'utilisation de dérivés d'oxazolidinone en particulier comme agents anti-pénétrants, dans une composition à application topique destinée notamment à traiter la peau du visage et/ou du corps humain, y compris le cuir chevelu, les cheveux et/ou les muqueuses.

Il est connu d'introduire dans les compositions cosmétiques et/ou dermatologiques des actifs en vue d'apporter des traitements spécifiques à la peau, aux muqueuses ou aux cheveux, par exemple pour lutter contre le dessèchement, le vieillissement ou la pigmentation de la peau, pour traiter l'acné ou certaines maladies de la peau (eczéma, psoriasis), pour combattre les surcharges pondérales, pour favoriser la restructuration de la peau ou son renouvellement cellulaire, pour protéger la peau ou les cheveux des rayons du soleil, ou aussi pour nettoyer ou colorer la peau ou les cheveux, ou encore pour maquiller la peau.

Malheureusement, certains actifs présentent l'inconvénient de provoquer des picotements, des démangeaisons, des tiraillements après leur application, pouvant conduire à un important inconfort. L'utilisation de ces composés pour les utilisateurs à peau sensible est donc souvent rédhibitoire.

Pour remédier à ces inconvénients, un certain nombre de solutions ont été proposées. En particulier, il est prévu dans le document WO-A-93/21904 l'utilisation d'un polyuréthanne hydrosoluble pour diminuer l'irritation provoquée par l'acide rétinoïque. Par ailleurs, il est connu d'utiliser certains dérivés polymères tels que la polyvinylpyrrolidone (voir FR-A-2224126) ou les polysiloxanes polyoxyéthylénés (voir EP-A-266921) pour diminuer l'irritation de la peau. En outre, il est connu du document EP-A-391274 d'utiliser des codérivés polymères acryliques en solution pour éviter la pénétration de certains produits irritants.

La demanderesse a trouvé de façon surprenante que certains dérivés d'oxazolidinone présentaient la propriété de réduire voire supprimer la pénétration d'actifs tout en ne diminuant pas leur efficacité. Cette propriété peut être mise à profit pour diminuer voire supprimer les effets secondaires indésirables apportés par ces actifs, notamment les actifs à effet secondaire irritant ou sensibilisant, c'est-à-dire possédant un potentiel de risques secondaires. Cette propriété est d'autant plus surprenante qu'elle va à l'encontre de ce qui est décrit dans l'art antérieur. En effet, le document US-A-4960771 décrit l'utilisation de dérivés d'oxazolidinone pour améliorer la pénétration d'agents physiologiquement actifs.

L'invention a pour objet l'utilisation d'au moins un dérivé d'oxazolidinone de formule (I) : 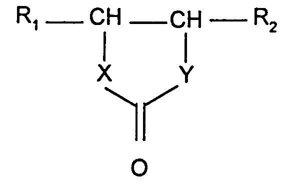 dans laquelle :
R₁ représente un radical hydroxyalkyle linéaire ou ramifié, saturé ou insaturé ayant de 6 à 27 atomes de carbone,
R₂ représente l'hydrogène ou un radical alkyle ou hydroxyalkyle linéaire ou ramifié, saturé ou insaturé ayant de 1 à 7 atomes de carbone,
Y et X, différents l'un de l'autre, représentent -NR₃ ou un atome d'oxygène, R₃ représentant l'hydrogène ou un radical alkyle ou hydroxyalkyle linéaire ou ramifié, saturé ou insaturé ayant de 1 à 8 atomes de carbone,
   dans une composition cosmétique ou pour la fabrication d'une composition dermatologique contenant un milieu physiologiquement acceptable, pour diminuer ou empêcher la pénétration d'au moins un actif compatible avec la peau, les cheveux et/ou les muqueuses.

Quand l'actif peut être potentiellement irritant ou sensibilisant, la présence d'un dérivé d'oxazolidinone selon l'invention permet d'empêcher la pénétration de cet actif et par là même l'irritation et/ou la sensibilisation de la composition le contenant, sans diminuer l'efficacité de l'actif. On peut ainsi obtenir une composition non irritante, applicable par voie topique sur la peau du visage et/ou du corps humain, les cheveux et/ou les muqueuses.

Aussi, la présente invention a encore pour objet l'utilisation d'au moins un dérivé d'oxazolidinone de formule (I) : 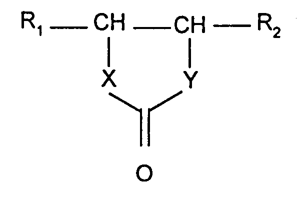 dans laquelle :
R₁ représente un radical hydroxyalkyle linéaire ou ramifié, saturé ou insaturé ayant de 6 à 27 atomes de carbone,
R₂ représente l'hydrogène ou un radical alkyle ou hydroxyalkyle linéaire ou ramifié, saturé ou insaturé ayant de 1 à 7 atomes de carbone,
Y et X, différents l'un de l'autre, représentent -NR₃ ou un atome d'oxygène, R₃ représentant l'hydrogène ou un radical alkyle ou hydroxyalkyle linéaire ou ramifié, saturé ou insaturé ayant de 1 à 8 atomes de carbone,
   dans une composition cosmétique ou pour la fabrication d'une composition dermatologique contenant un milieu physiologiquement acceptable, pour diminuer ou empêcher l'apparition du caractère irritant ou sensibilisant d'au moins un actif ayant un effet secondaire irritant ou sensibilisant.

L'actif peut se trouver soit dans la même composition que le dérivé de formule (I) soit dans une autre composition appliquée en même temps ou après application de la composition contenant le dérivé de formule (I) ou encore juste avant l'application. De préférence, l'actif et le dérivé de formule (I) se trouvent dans la même composition.

Les dérivés de formule (I) peuvent être préparés de manière classique par réaction de carbonate d'éthylène sur un amino-alcane-polyol à une température supérieure à 60°C. La synthèse peut conduire à l'obtention de plusieurs stéréoisomères et isomères de position.

Les dérivés de formule (I) préférés sont ceux où, dans la formule (I) X représente -NH-, Y représente l'oxygène, R₁ représente un radical hydroxyalkyle saturé ayant de 12 à 20 atomes de carbone et R₂ représente l'hydrogène ou un radical alkyle ou hydroxyalkyle saturé ayant de 1 à 3 atomes de carbone.

Comme dérivés de formule (I) préférés, on peut citer par exemple la 4-(1-hydroxy-hexadécyl)-oxazolidine-2-one, la 4-pentadécyl-5-hydroxyméthyl-oxazolidine-2-one, la 4-(1,2-dihydroxy-hexadécyl)-oxazolidine-2-one, la 4-(1hyd roxy-pentadécyl)-5-hyd roxyméthyl-oxazolid ine-2-one, leurs stéréoisomères et leurs mélanges.

La composition contenant le dérivé de formule (I) comporte un milieu physiologiquement acceptable, c'est-à-dire compatible avec la peau y compris le cuir chevelu, les cheveux et/ou les muqueuses.

L'invention s'applique à tous les actifs compatibles avec la peau, les cheveux et/ou les muqueuses. A titre d'exemple d'actifs auxquels s'applique plus particulièrement l'invention, on peut citer les actifs exfoliants, c'est-à-dire des actifs ayant en particulier une propriété kératolytique et/ou comédolytique, les filtres solaires, les matières colorantes, les conservateurs, les tensioactifs, les parfums.

Comme actifs, on peut citer plus particulièrement les α-hydroxy-acides (acides glycolique, lactique, malique, citrique, tartrique, mandélique), les β-hydroxy-acides (acide salicylique et ses dérivés tels que les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique), les α-céto-acides, les β-céto-acides, les rétinoïdes (rétinol, acide rétinoïque, rétinal et leurs dérivés), les anthralines (dioxyanthranol), les anthranoïdes, les peroxydes (notamment de benzoyle), le minoxidil, les antimétabolites comme le fluoro-5-uracile, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les dépigmentants (hydroquinone), les pigments et les filtres chimiques.

Ces actifs peuvent selon la quantité utilisée et selon la sensibilité de la peau de l'utilisateur, se révéler plus ou moins irritants ou sensibilisants.

La quantité de dérivé d'oxazolidinone de formule (I) dans la composition peut aller par exemple de 0,05 à 20 %, et plus particulièrement de 0,1 à 5 % du poids total de la composition. En présence d'actif, cette quantité dépend en particulier de la quantité d'actif dans la composition.

Par ailleurs, la quantité d'actif peut varier dans une large mesure en fonction de la nature et/ou de la fonction de l'actif. Cette quantité peut aller par exemple de 0,001 à 20 % du poids total de la composition.

Les compositions contenant le dérivé d'oxazolidinone de formule (I) et/ou l'actif peuvent se présenter sous toutes les formes galéniques normalement appropriées pour une application topique, et par exemple sous forme d'une solution aqueuse, alcoolique, hydroalcoolique ou huileuse, d'un gel aqueux ou huileux, d'une composition solide anhydre, d'une dispersion du type lotion ou sérum, d'une émulsion du type eau-dans-huile (E/H), huile-dans-eau (H/E) ou multiple (E/H/E ou H/E/H), d'une microémulsion ou d'une dispersion de vésicules de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants desdites compositions sont celles classiquement utilisées dans les domaines considérés.

Ces compositions constituent notamment des crèmes de nettoyage, de protection, de traitement ou de soin pour le visage, pour les mains, pour les pieds ou pour le corps, (par exemple crèmes de jour, crèmes de nuit, crèmes démaquillantes, crèmes de fond de teint, crèmes solaires), des produits de maquillage comme des fonds de teint, des fards à joues ou des sticks pour les lèvres, des laits de démaquillage, des laits corporels de protection ou de soin, des laits solaires, des lotions, gels ou mousses pour le soin de la peau, comme des lotions de nettoyage, des lotions solaires, des lotions de bronzage artificiel, des compositions pour le bain, des compositions désodorisantes contenant un agent bactéricide, des gels ou lotions après-rasage, des crèmes épilatoires, des compositions contre les piqûres d'insectes, des compositions anti-douleur.

Le ou les dérivés de formule (I) peuvent être aussi incorporés dans diverses compositions pour soins capillaires, et notamment des shampooings éventuellement antiparasitaires, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, etc.

Lesdites compositions peuvent aussi être à usage bucco-dentaire, par exemple une pâte dentifrice. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lesdites compositions peuvent également consister en des préparations solides constituant des savons ou des pains de nettoyage.

Les compositions peuvent aussi être conditionnées sous forme de composition pour aérosol contenant également un agent propulseur sous pression.

Lorsque ladite composition est une émulsion, la proportion de la phase grasse peut aller de 5 à 80 %, et de préférence de 5 à 50 % du poids total de la composition. Les matières grasses, les émulsionnants et les coémulsionnants utilisés dans la composition sous forme d'émulsion sont choisis parmi ceux classiquement utilisés dans les domaines cosmétique et dermatologique. L'émulsionnant et le coémulsionnant sont présents, dans la composition, en une proportion allant de 0,3 à 30 %, et de préférence de 0,5 à 20 % du poids total de la composition. L'émulsion peut, en outre, contenir des vésicules lipidiques.

De façon connue, ladite composition peut contenir également des adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans les domaines considérés, et par exemple de 0,01 à 30 % du poids total de la composition. Ces adjuvants, selon leur nature, peuvent être introduits dans la phase grasse, dans la phase aqueuse et/ou dans les sphérules lipidiques.

Comme matières grasses utilisables dans l'invention, on peut citer les huiles et plus particulièrement les huiles minérales, les huiles d'origine végétale, les huiles d'origine animale, les huiles de synthèse, les huiles siliconées et les huiles fluorées. On peut aussi utiliser comme matières grasses des alcools gras, des acides gras, des cires.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

D'autres caractéristiques et avantages de l'invention ressortiront mieux des exemples qui suivent, donnés à titre illustratif.

L'exemple suivant illustre le procédé de préparation de dérivés utilisables selon l'invention.

### Exemple 1 : Préparation du mélange de 4-(1 -hydroxy-hexadécyl)-oxazolidine-2-one et de 4-pentadécyl-5-hydroxyméthyl-oxazolidine-2-one

On fait réagir 10 g de 2-amino-1 ,3-octadécanediol (3,31 x 10⁻² mole) avec 3,2 g de carbonate d'éthylène (3,64 x 10⁻² mole) à 110 °C pendant 48 heures. Le mélange est ensuite recristallisé dans 200 ml d'heptane. On obtient 10,2 g (rendement : 94 %) d'un mélange de 4-(1-hydroxy-hexadécyl)-oxazolidine-2-one et de 4-pentadécyl-5-hydroxyméthyl-oxazolidine-2-one sous forme d'une poudre blanche ayant un point de fusion de 79°C.

| Analyse élémentaire | | | | |
|---|---|---|---|---|
| | C | H | N | O |
| Calculé | 69,68 | 11,39 | 4,28 | 14,66 |
| Trouvé | 69,14 | 11,57 | 4,21 | 15,13 |

Le RMN ¹H et la masse du produit obtenu sont conformes à la structure attendue.

### Exemple 2 : Test

L'activité anti-pénétrante des dérivés de formule (I) a été évaluée sur un modèle de membrane cellulaire constituée de dipalmitoyl-phosphatidylcholine hydraté. Une augmentation de la température de transition de phase de la membrane reflète une augmentation de la cohésion de la membrane, entraînant une moindre pénétration de cette dernière par des actifs.

Le tableau 1 rapporte les valeurs des températures de transition de phase du dipalmitoyl-phosphatidylcholine (D.P.P.C.) seul, comparées à celles de ce même lipide associé à un mélange de deux dérivés isomères de formule (I), correspondant à l'exemple 1, c'est-à-dire la 4-(1-hydroxy-hexadécyl)-oxazolidine-2-one et son isomère de position, la 4-pentadécyl-5-hydroxyméthyl-oxazolidine-2-one. Les compositions sont mises à 50 % dans l'eau et caractérisés par analyse thermique différentielle avec chauffage entre 1 °C et 110 °C à raison de 2°C/mn.

**Tableau 1**

| Compositions molaires | Températures de transition en °C | |
|---|---|---|
| | début | maximum |
| D.P.P.C. | 40,6 ± 0,1 | 41,8 ± 0,1 |
| D.P.P.C./mélange de l'exemple 1 (90/10) | 41,5 ± 0,1 | 42,9 ± 0,1 |
| D.P.P.C./mélange de l'exemple 1 (75/25) | 45,0 ± 0,2 | 47 ± 0,4 |
| D.P.P.C./mélange de l'exemple 1 (50/50) | 48,2 ± 0,2 | 49,4 ± 0,3 |

Les résultats du tableau 1 montrent une nette augmentation des températures de transition de phase (début et maximum) du dipalmitoyl-phosphatidylcholine quand il est associé aux composés selon l'invention, ce qui met en évidence une moindre pénétration de la membrane cellulaire que constitue le dipalmitoyl-phosphatidylcholine.

Dans la même étude, la N-dodécyl-ε-caprolactame (azone) et la 4-décyloxazolidine-2-one (exemple 1 du brevet US-4960771), à l'inverse du mélange de l'exemple 1 de notre demande, abaissent la température de transition de phase de la D.P.P.C. et ceci, de façon significative (températures de transition de phase au maximum inférieures à 40 °C) pour des mélanges D.P.P.C à 50 % en association avec l'un ou l'autre de ces composés (voir tableau 2).

**Tableau 2**

| Compositions molaires | Températures de transition en °C | |
|---|---|---|
| | début | maximum |
| D.P.P.C. | 40,6 ± 0,1 | 41,8 ± 0,1 |
| D.P.P.C./N-dodécyl-ε-caprolactame (50/50) | 15,6 ± 0,4 | 30,1 ± 0,0 |
| D.P.P.C./4-décyl-oxazolidine-2-one (50/50) | 10,4 ± 0,0 | 29,4 ± 0,2 |

Ces résultats montrent que les dérivés de l'art antérieur abaissent la température de transition de phase et sont inaptes à empêcher la pénétration d'actifs.

Les exemples suivants illustrent lesdites compositions cosmétiques ou dermatologiques. Les quantités sont données en pourcentage en poids.

### Exemple 3 : dispersion vésiculaire

| | |
|---|---|
| - mélange de 4-(1-hydroxy-hexadécyl)-oxazolidine-2-one et de 4-pentadécyl-5-hydroxyméthyl-oxazolidine-2-one | 2,14 % |
| - cholestérol | 2,14% |
| - dicétylphosphate de sodium | 0,48 % |
| - conservateur | 0,24 % |
| - glycérol (hydratant) | 2,91 % |
| - eau qsp | 100 % |

La dispersion est obtenue par dissolution des lipides (mélange de 4-(1-hydroxyhexadécyl)-oxazolidine-2-one et de 4-pentadécyl-5-hydroxyméthyl-oxazolidine-2-one + cholestérol + dicétylphosphate de sodium) dans du solvant (CH₂Cl₂), évaporation du solvant et dispersion des lipides dans la phase aqueuse contenant l'eau, le glycérol et le conservateur.

On obtient une dispersion empêchant la pénétration d'actif lors de l'application sur la peau.

### Exemple 4 : Composition de soin contenant une dispersion vésiculaire

| | |
|---|---|
| - mélange de 4-(1-hydroxy-hexadécyl)-oxazolidine-2-one et de 4-pentadécyl-5-hydroxyméthyl-oxazolidine-2-one | 2,25 % |
| - cholestérol | 2,24% |
| - dicétylphosphate de sodium | 0,50 % |
| -conservateur | 0,24 % |
| - acide lactique | 1,91 % |
| - eau qsp | 100 % |

La dispersion est obtenue par dissolution des lipides (mélange de 4-(1-hydroxy-hexadécyl)-oxazolidine-2-one et de 4-pentadécyl-5-hydroxyméthyl-oxazolidine-2-one + cholestérol + dicétylphosphate de sodium) dans du solvant (CH₂Cl₂), évaporation du solvant et dispersion des lipides dans la phase aqueuse contenant l'eau, l'acide lactique et le conservateur.

On obtient une dispersion apte à lisser la peau sans l'irriter.

## Revendications

1. Utilisation d'au moins un dérivé d'oxazolidinone de formule (I) : 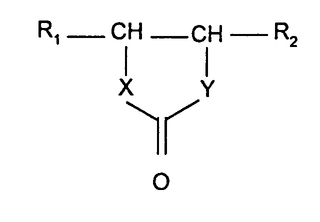 dans laquelle :
R₁ représente un radical hydroxyalkyle linéaire ou ramifié, saturé ou insaturé ayant de 6 à 27 atomes de carbone,
R₂ représente l'hydrogène ou un radical alkyle ou hydroxyalkyle linéaire ou ramifié, saturé ou insaturé ayant de 1 à 7 atomes de carbone,
Y et X, différents l'un de l'autre, représentent -NR₃ ou un atome d'oxygène, R₃ représentant l'hydrogène ou un radical alkyle ou hydroxyalkyle linéaire ou ramifié, saturé ou insaturé ayant de 1 à 8 atomes de carbone,
dans une composition cosmétique ou pour la fabrication d'une composition dermatologique contenant un milieu physiologiquement acceptable, pour diminuer ou empêcher la pénétration d'au moins un actif compatible avec la peau, les cheveux et/ou les muqueuses.

2. Utilisation d'au moins un dérivé d'oxazolidinone de formule (I) : 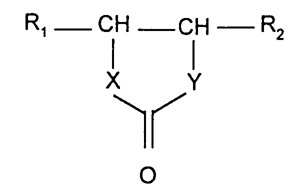 dans laquelle :
R₁ représente un radical hydroxyalkyle linéaire ou ramifié, saturé ou insaturé ayant de 6 à 27 atomes de carbone,
R₂ représente l'hydrogène ou un radical alkyle ou hydroxyalkyle linéaire ou ramifié, saturé ou insaturé ayant de 1 à 7 atomes de carbone,
Y et X, différents l'un de l'autre, représentent -NR₃ ou un atome d'oxygène, R₃ représentant l'hydrogène ou un radical alkyle ou hydroxyalkyle linéaire ou ramifié, saturé ou insaturé ayant de 1 à 8 atomes de carbone,
dans une composition cosmétique ou pour la fabrication d'une composition dermatologique contenant un milieu physiologiquement acceptable, pour diminuer ou empêcher l'apparition du caractère irritant ou sensibilisant d'au moins un actif ayant un effet secondaire irritant ou sensibilisant.

3. Utilisation selon la revendication 1 ou 2, caractérisée en ce que, dans la formule (I), X représente -NH-, Y représente l'oxygène, R₁ représente un radical hydroxyalkyle saturé ayant de 12 à 20 atomes de carbone et R₂ représente l'hydrogène ou un radical alkyle ou hydroxyalkyle saturé ayant de 1 à 3 atomes de carbone.

4. Utilisation selon la revendication précédente, caractérisée en ce que les dérivés de formule (I) sont choisis dans le groupe comprenant la 4-(1-hydroxy-hexadécyl)-oxazolidine-2-one, la 4-pentadécyl-5-hydroxyméthyl-oxazolidine-2-one, la 4-(1,2-dihydroxy-hexadécyl)-oxazolidine-2-one, la 4-(1-hydroxy-pentadécyl)-5-hydroxyméthyl-oxazolidine-2-one, leurs stéréoisomères et leurs mélanges.

5. Utilisation selon l'une quelconque des revendications précédentes, caractérisée en ce que le dérivé de formule (I) et l'actif sont dans des compositions distinctes.

6. Utilisation selon l'une quelconque des revendications 1 à 4, caractérisée en ce que le dérivé de formule (I) et l'actif sont dans la même composition.

7. Utilisation selon l'une quelconque des revendications précédentes, caractérisée en ce que l'actif est choisi parmi les actifs ayant une propriété kératolytique et/ou comédolytique, les filtres solaires, les matières colorantes, les conservateurs, les tensioactifs, les parfums.

8. Utilisation selon l'une quelconque des revendications précédentes, caractérisée en ce que l'actif est choisi parmi les α-hydroxy-acides, les β-hydroxy-acides, les α- céto-acides, les β-céto-acides, les rétinoïdes, l'anthraline, les anthranoïdes, les peroxydes, le minoxidil, les antimétabolites, les teintures ou colorants capillaires, les solutions alcooliques parfumantes, les agents antitranspirants, les actifs dépilatoires ou de permanentes, les dépigmentants, les pigments et les filtres chimiques.

9. Utilisation selon l'une quelconque des revendications précédentes, caractérisée en ce que le dérivé de formule (I) est présent en une quantité allant de 0,05 à 20 % du poids total de la composition.

10. Utilisation selon l'une quelconque des revendications précédentes, caractérisée en ce que le dérivé de formule (I) est présent en une quantité allant de 0,1 à 5 % du poids total de la composition.

11. Utilisation selon l'une quelconque des revendications précédentes, caractérisée en ce que l'actif est présent en une quantité allant de 0,001 à 20 % du poids total de la composition.

12. Utilisation selon l'une quelconque des revendications précédentes, caractérisée en ce que la composition se présente sous forme d'une solution, d'un gel, d'une dispersion, d'une émulsion, d'une microémulsion ou d'une dispersion de vésicules de type ionique et/ou non ionique.

13. Utilisation selon l'une quelconque des revendications précédentes, caractérisée en ce que la composition constitue une composition de nettoyage, de protection, de traitement, de soin ou de maquillage de la peau et/ou des cheveux.

## Claims

1. Use of at least one oxazolidinone derivative of formula (I): 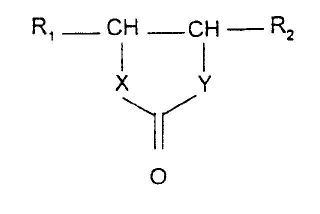 in which:
R₁ represents a linear or branched, saturated or unsaturated hydroxyalkyl radical having from 6 to 27 carbon atoms,
R₂ represents hydrogen or a linear or branched, saturated or unsaturated alkyl or hydroxyalkyl radical having from 1 to 7 carbon atoms,
X and Y, which are different from each other, represent -NR₃ or an oxygen atom, R₃ representing hydrogen or a linear or branched, saturated or unsaturated alkyl or hydroxyalkyl radical having from 1 to 8 carbon atoms,
in a cosmetic composition or for the manufacture of a dermatological composition containing a physiologically acceptable medium, in order to reduce or prevent the penetration of at least one active agent which is compatible with the skin, the hair and/or mucous membranes.

2. Use of at least one oxazolidinone derivative of formula (I): 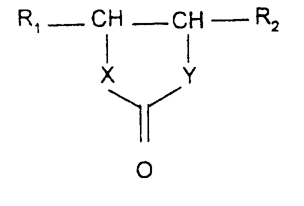 in which:
R₁ represents a linear or branched, saturated or unsaturated hydroxyalkyl radical having from 6 to 27 carbon atoms,
R₂ represents hydrogen or a linear or branched, saturated or unsaturated alkyl or hydroxyalkyl radical having from 1 to 7 carbon atoms,
X and Y, which are different from each other, represent -NR₃ or an oxygen atom, R₃ representing hydrogen or a linear or branched, saturated or unsaturated alkyl or hydroxyalkyl radical having from 1 to 8 carbon atoms,
in a cosmetic composition or for the manufacture of a dermatological composition containing a physiologically acceptable medium, in order to reduce or prevent the appearance of the irritant or sensitizing nature of at least one active agent having an irritant or sensitizing side effect.

3. Use according to Claim 1 or 2, characterized in that, in formula (I), X represents -NH-, Y represents oxygen, R₁ represents a saturated hydroxyalkyl radical having from 12 to 20 carbon atoms and R₂ represents hydrogen or a saturated alkyl or hydroxyalkyl radical having from 1 to 3 carbon atoms.

4. Use according to the preceding claim, characterized in that the derivatives of formula (I) are chosen from the group comprising 4-(1-hydroxyhexadecyl)-2-oxazolidinone, 4-pentadecyl-5-hydroxymethyl-2-oxazolidinone, 4-(1,2-dihydroxyhexadecyl)-2-oxazolidinone, 4-(1-hydroxypentadecyl)-5-hydroxymethyl-2-oxazolidinone, their stereoisomers and their mixtures.

5. Use according to any one of the preceding claims, characterized in that the derivative of formula (I) and the active agent are in separate compositions.

6. Use according to any one of Claims 1 to 4, characterized in that the derivative of formula (I) and the active agent are in the same composition.

7. Use according to any one of the preceding claims, characterized in that the active agent is chosen from active agents having a keratolytic and/or comedolytic property, sunscreens, dyestuffs, preserving agents, surfactants and fragrances.

8. Use according to any one of the preceding claims, characterized in that the active agent is chosen from α-hydroxy acids, β-hydroxy acids, α-keto acids, β-keto acids, retinoids, anthralin, anthranoids, peroxides, Minoxidil, antimetabolites, hair tints or dyes, perfuming alcoholic solutions, antiperspirants, hair-removing or permanent-waving active agents, depigmenting agents, pigments and chemical screening agents.

9. Use according to any one of the preceding claims, characterized in that the derivative of formula (I) is present in an amount ranging from 0.05 to 20% of the total weight of the composition.

10. Use according to any one of the preceding claims, characterized in that the derivative of formula (I) is present in an amount ranging from 0.1 to 5% of the total weight of the composition.

11. Use according to any one of the preceding claims, characterized in that the active agent is present in an amount ranging from 0.001 to 20% of the total weight of the composition.

12. Use according to any one of the preceding claims, characterized in that the composition is in the form of a solution, a gel, a dispersion, an emulsion, a microemulsion or a vesicle dispersion of ionic and/or nonionic type.

13. Use according to any one of the preceding claims, characterized in that the composition constitutes a cleansing, protective, treatment, care or make-up composition for the skin and/or the hair.

## Patentansprüche

1. Verwendung mindestens eines Oxazolidinon-Derivats der Formel (I) 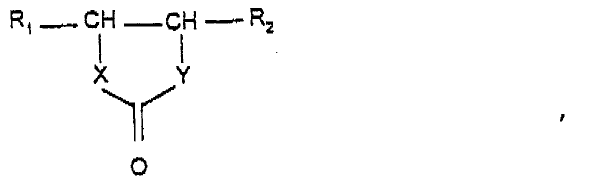 in der bedeuten:
R₁ geradkettiges oder verzweigtes, gesättigtes oder ungesättigtes Hydroxyalkyl mit 6 bis 27 Kohlenstoffatomen,
R₂ Wasserstoff oder geradkettiges oder verzweigtes, gesättigtes oder ungesättigtes Alkyl oder Hydroxyalkyl mit 1 bis 7 Kohlenstoffatomen,
Y und X, die verschieden sind, -NR₃ oder ein Sauerstoffatom, wobei R₃ Wasserstoff oder geradkettiges oder verzweigtes, gesättigtes oder ungesättigtes Alkyl oder Hydroxyalkyl mit 1 bis 8 Kohlenstoffatomen darstellt,
in einer kosmetischen Zusammensetzung oder für die Herstellung einer dermatologischen Zusammensetzung, die ein physiologisch akzeptables Medium enthält, zur Verringerung oder Verhinderung der Penetration mindestens eines Wirkstoffs, der mit der Haut, den Haaren und/oder den Schleimhäuten verträglich ist.

2. Verwendung mindestens eines Oxazolidinon-Derivats der Formel (I) 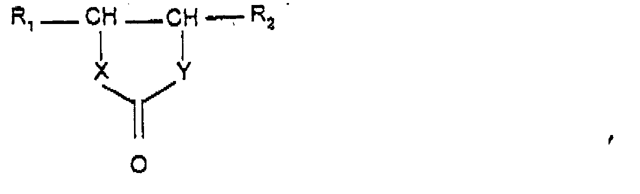 in der bedeuten:
R₁ geradkettiges oder verzweigtes, gesättigtes oder ungesättigtes Hydroxyalkyl mit 6 bis 27 Kohlenstoffatomen,
R₂ Wasserstoff oder geradkettiges oder verzweigtes, gesättigtes oder ungesättigtes Alkyl oder Hydroxyalkyl mit 1 bis 7 Kohlenstoffatomen,
Y und X, die verschieden sind, -NR₃ oder ein Sauerstoffatom, wobei R₃ Wasserstoff oder geradkettiges oder verzweigtes, gesättigtes oder ungesättigtes Alkyl oder Hydroxyalkyl mit 1 bis 8 Kohlenstoffatomen darstellt,
in einer kosmetischen Zusammensetzung oder für die Herstellung einer dermatologischen Zusammensetzung, die ein physiologisch akzeptables Medium enthält, zur Verringerung oder Verhinderung des Auftretens der reizenden oder sensibilisierenden Wirkung mindestens eines Wirkstoffs, der eine reizende oder sensibilisierende Nebenwirkung aufweist.

3. Verwendung nach Anspruch 1 oder 2, dadurch gekennzeichnet, daß in der Formel (I) X -NH-, Y Sauerstoff, R₁ gesättigtes Hydroxyalkyl mit 12 bis 20 Kohlenstoffatomen und R₂ Wasserstoff oder gesättigtes Alkyl oder Hydroxyalkyl mit 1 bis 3 Kohlenstoffatomen darstellt.

4. Verwendung nach dem vorhergehenden Anspruch, dadurch gekennzeichnet, daß die Derivate der Formel (I) unter 4-(1-Hydroxyhexadecyl)-oxazolidin-2-on, 4-Pentadecyl-5-hydroxymethyloxazolidin-2-on, 4-(1,2-Dihydroxyhexadecyl)-oxazolidin-2-on, 4-(1-Hydroxypentadecyl)-5-hydroxymethyloxazolidin-2-on, ihren Stereoisomeren und ihren Gemischen ausgewählt werden.

5. Verwendung nach einem der vorhergehenden Ansprüche, dadurch gekennzeichnet, daß das Derivat der Formel (I) und der Wirkstoff in verschiedenen Zusammensetzungen enthalten sind.

6. Verwendung nach einem der Ansprüche 1 bis 4, dadurch gekennzeichnet, daß das Derivat der Formel (I) und der Wirkstoff in der gleichen Zusammensetzung enthalten sind.

7. Verwendung nach einem der vorhergehenden Ansprüche, dadurch gekennzeichnet, daß der Wirkstoff unter den Wirkstoffen, die keratolytische und/oder komedolytische Eigenschaften aufweisen, Sonnenschutzfiltern, Färbemitteln, Konservierungsmitteln, grenzflächenaktiven Stoffen, Parfums ausgewählt wird.

8. Verwendung nach einem der vorhergehenden Ansprüche, dadurch gekennzeichnet, daß der Wirkstoff unter α-Hydroxysäuren, β-Hydroxysäuren, α-Ketosäuren, β-Ketosäuren, Retinoiden, Anthralin, Anthranoiden, Peroxiden, Minoxidil, Antimetaboliten, Haarfarben oder Haarfärbemitteln, parfümierenden alkoholischen Lösungen, Antitranspirantien, haarentfernenden Wirkstoffen und Wirkstoffen für die Dauerwellbehandlung, Depigmentierungsmitteln, Pigmenten und chemischen Filtern ausgewählt wird.

9. Verwendung nach einem der vorhergehenden Ansprüche, dadurch gekennzeichnet, daß das Derivat der Formel (I) in einem Mengenanteil von 0,05 bis 20 % des Gesamtgewichts der Zusammensetzung enthalten ist.

10. Verwendung nach einem der vorhergehenden Ansprüche, dadurch gekennzeichnet, daß das Derivat der Formel (I) in einem Mengenanteil von 0,1 bis 5 % des Gesamtgewichts der Zusammensetzung enthalten ist.

11. Verwendung nach einem der vorhergehenden Ansprüche, dadurch gekennzeichnet, daß der Wirkstoff in einem Mengenanteil von 0,001 bis 20 % des Gesamtgewichts der Zusammensetzung enthalten ist.

12. Verwendung nach einem der vorhergehenden Ansprüche, dadurch gekennzeichnet, daß die Zusammensetzung in Form einer Lösung, eines Gels, einer Dispersion, einer Emulsion, einer Mikroemulsion oder einer Vesikeldispersion vom Typ der ionischen und/oder nichtionischen Vesikeldispersionen vorliegt.

13. Verwendung nach einem der vorhergehenden Ansprüche, dadurch gekennzeichnet, daß die Zusammensetzung eine Zusammensetzung zur Reinigung, zum Schutz, zur Behandlung, zur Pflege oder zum Schminken der Haut und/oder der Schleimhäute ist.
